# EUROPÄISCHE PATENTSCHRIFT

(11) **EP 4 139 214 B1**
(45) Veröffentlichungstag und Bekanntmachung des Hinweises auf die Patenterteilung: **01.05.2024**
(21) Anmeldenummer: 21721010.3
(22) Anmeldetag: 19.04.2021
(51) Int. Cl.: B65B 55/16, A61L 2/08

(54) **VORRICHTUNG UND VERFAHREN ZUR ERZEUGUNG EINES FLÜSSIGKEITSFILMS EINES FLÜSSIGEN MEDIUMS IN EINEM FOLIENBEUTEL, SOWIE ANORDNUNG ZUR KONTROLLIERTEN EXPOSITION EINES FLÜSSIGEN MEDIUMS IN EINEM FOLIENBEUTEL MIT PHYSIKALISCHER STRAHLUNG**
DEVICE AND METHOD FOR GENERATING A LIQUID FILM OF A LIQUID MEDIUM IN A FILM BAG, AND ASSEMBLY FOR THE CONTROLLED EXPOSURE OF A LIQUID MEDIUM IN A FILM BAG USING PHYSICAL RADIATION
DISPOSITIF ET PROCÉDÉ PERMETTANT DE GÉNÉRER UN FILM LIQUIDE D'UN MILIEU LIQUIDE DANS UN SAC EN PELLICULE ET ENSEMBLE POUR L'EXPOSITION CONTRÔLÉE D'UN MILIEU LIQUIDE DANS UN SAC EN PELLICULE À L'AIDE D'UN RAYONNEMENT PHYSIQUE

(30) Priorität: 21.04.2020 DE 102020205036
(43) Veröffentlichungstag der Anmeldung: 01.03.2023
(73) Patentinhaber: Fraunhofer-Gesellschaft zur Förderung der angewandten Forschung e.V., 80686 München (DE)
(72) Erfinder: THOMA, Martin, 70569 Stuttgart (DE); STANDFEST, Bastian, 70569 Stuttgart (DE); KLINGER, Michael, 70569 Stuttgart (DE)
(74) Vertreter: Kordel, Mattias
(86) Internationale Anmeldenummer: PCT/EP2021/060075
(87) Internationale Veröffentlichungsnummer: WO 2021/213973

(56) Entgegenhaltungen:
- DE-B3-102015 224 206
- DE-C1- 4 209 509
- US-A- 4 866 282
- US-B1- 6 369 394

## Beschreibung

Die Erfindung betrifft eine Vorrichtung zur Erzeugung eines Flüssigkeitsfilms eines flüssigen Mediums in einem Folienbeutel, eine Anordnung zur kontrollierten Exposition eines flüssigen Mediums in einem Folienbeutel mit physikalischer Strahlung mit einer solchen Vorrichtung, und ein Verfahren zum Erzeugen eines Flüssigkeitsfilms eines flüssigen Mediums in einem Folienbeutel.

Moderne Verfahren zur Bereitstellung und Verarbeitung flüssiger Medien, insbesondere flüssiger biologischer Medien, sehen häufig eine kontrollierte Exposition eines solchen flüssigen Mediums mit physikalischer Strahlung vor, beispielsweise zur Sterilisation oder Desinfektion, zur Haltbarmachung, zur Inaktivierung von Zellenproliferation, oder um enthaltene Viren zu inaktivieren. Um die auf das flüssige Medium einwirkende Strahlendosis genau kontrollieren zu können, ist es insbesondere wichtig, dass ein bestrahlter Flüssigkeitsfilm mit homogener und zeitlich konstanter Schichtdicke erzeugt wird. Beispielsweise verlieren bei einer ElektronenBestrahlung die beschleunigten Elektronen mit zunehmender Eindringtiefe an Energie, sodass die Tiefendosis abnimmt. Die Schichtdicke einerseits und auch die Fluidgeschwindigkeit des flüssigen Mediums im Bereich des bestrahlten Flüssigkeitsfilms andererseits haben jeweils einen direkten Einfluss auf die applizierte Dosis. Sollen flüssige Medien automatisiert bestrahlt werden, bedarf es daher eines gleichmäßigen, regelbaren Flüssigkeitsfilms, um eine homogene Behandlung zu gewährleisten.

Aus dem deutschen Patent DE 10 2015 224 206 B3 geht eine Vorrichtung zur Erzeugung eines solchen Flüssigkeitsfilms hervor, wobei ein Folienbeutel zwischen zwei Führungsplatten geklemmt und mittels eines Reibrads gespannt und transportiert wird. Während des Transports des Folienbeutels nimmt eine Reibfläche zwischen dem Folienbeutel und den Führungsplatten ab, wodurch auch die Beutelspannung abnimmt. Dies führt zu einer Inhomogenität der Schichtdicke im Folienbeutel. Darüber hinaus ermöglicht die Vorrichtung keine schichtdickenabhängige Regelung der Beutelspannung oder der Transportgeschwindigkeit. Insbesondere sind Beutelspannung einerseits und Transport des Folienbeutels andererseits miteinander gekoppelt und können nicht unabhängig voneinander eingestellt werden. Dies ist insbesondere mit Blick auf reproduzierbare Bestrahlungsergebnisse sowie das kontrollierte Einbringen einer bestimmten Strahlendosis verbesserungsfähig.

Der Erfindung liegt daher die Aufgabe zugrunde, eine Vorrichtung zur Erzeugung eines Flüssigkeitsfilms eines flüssigen Mediums in einem Folienbeutel, eine Anordnung zur kontrollierten Exposition des flüssigen Mediums in dem Folienbeutel mit physikalischer Strahlung sowie ein Verfahren zum Erzeugen eines Flüssigkeitsfilms eines flüssigen Mediums in einem Folienbeutel zu schaffen, wobei die genannten Nachteile zumindest vermindert, vorzugsweise behoben sind.

Die Aufgabe wird gelöst, indem die vorliegende technische Lehre bereitgestellt wird, insbesondere die Lehre der unabhängigen Ansprüche sowie der in den abhängigen Ansprüchen und der Beschreibung offenbarten Ausführungsbeispiele.

Die Aufgabe wird insbesondere gelöst, indem eine Vorrichtung zur Erzeugung eines Flüssigkeitsfilms eines flüssigen Mediums in einem Folienbeutel bereitgestellt wird, die zwei voneinander durch einen Stauabstand beabstandete Stauflächen aufweist, zwischen denen der Folienbeutel angeordnet und entlang einer Transportrichtung transportiert werden kann. In Transportrichtung hinter den Stauflächen ist ein Bremselement angeordnet, das eingerichtet ist, um den Folienbeutel abzubremsen. Die Vorrichtung weist außerdem eine in Transportrichtung konvex gekrümmte Lauffläche auf, die derart eingerichtet und angeordnet ist, dass der durch das Bremselement gebremste Folienbeutel auf der Lauffläche hinter dem Bremselement aufgespannt werden kann. Zumindest eine erste Staufläche der beiden Stauflächen ist zumindest bereichsweise durch ein angetriebenes erstes Transportband gebildet, das eingerichtet ist, um den Folienbeutel in Transportrichtung zu fördern. Die Vorrichtung weist in Transportrichtung hinter dem Bremselement ein ansteuerbares Spannelement auf, das eingerichtet ist, um den Folienbeutel auf der Lauffläche zwischen dem Bremselement und dem Spannelement aufzuspannen. Das erste Transportband oder ein Bandantrieb für das erste Transportband ist unabhängig von dem ansteuerbaren Spannelement ansteuerbar. Insbesondere dadurch, dass die hier vorgeschlagene Vorrichtung einerseits das angetriebene erste Transportband für den Transport des Folienbeutels durch die Vorrichtung und andererseits das ansteuerbare Spannelement aufweist, um den Folienbeutel aufzuspannen, ist es möglich, die Beutelspannung des Folienbeutels einerseits und die Transportgeschwindigkeit durch die Vorrichtung andererseits unabhängig voneinander einzustellen, insbesondere zu regeln. Dies gewährleistet eine homogene sowie zeitlich konstante Schichtdicke und damit nicht zuletzt bei einer Beaufschlagung des Folienbeutels mit physikalischer Strahlung das Einbringen einer kontrollierten Dosis in das flüssige Medium.

Das ansteuerbare Spannelement ist dabei insbesondere ansteuerbar, um die Beutelspannung des Folienbeutels zu beeinflussen, insbesondere einzustellen, vorzugsweise zu regeln. Das angetriebene erste Transportband ist vorzugsweise ansteuerbar, oder der Bandantrieb für das erste angetriebene Transportband ist vorzugsweise ansteuerbar, um die Transportgeschwindigkeit des Folienbeutels durch die Vorrichtung einzustellen, vorzugsweise zu regeln.

Das erste Transportband oder der Bandantrieb für das erste Transportband ist unabhängig von dem ansteuerbaren Spannelement ansteuerbar, sodass eine Entkopplung der Einstellung, vorzugsweise Einregelung, der Beutelspannung von der Einstellung, vorzugsweise Einregelung, der Transportgeschwindigkeit für den Folienbeutel gegeben ist.

Nicht zuletzt kann bei der hier vorgeschlagenen Vorrichtung vorteilhaft vermieden werden, dass eine abnehmende Reibfläche zwischen dem Folienbeutel und den Stauflächen während des Transports dazu führt, dass die Beutelspannung abnimmt. Somit kann auch eine zeitlich zunehmende Inhomogenität der Schichtdicke im Folienbeutel vorteilhaft vermieden werden.

Soweit hier die Begriffe "vor" und "hinter" verwendet werden, sind diese insbesondere mit Bezug auf die Transportrichtung zu verstehen. Ein Element, das hinter einem anderen Element angeordnet ist, ist also in Transportrichtung hinter dem anderen Element angeordnet, sodass es beim Transport des Folienbeutels durch die Vorrichtung nach dem anderen Element erreicht wird. Entsprechend ist ein vor einem anderen Element angeordnetes Element in Transportrichtung vor diesem anderen Element angeordnet, sodass es beim Transport eines Folienbeutels durch die Vorrichtung zuerst, vor dem anderen Element, erreicht wird.

Unter einem Folienbeutel wird hier ein aus wenigstens einer Folie, insbesondere Kunststofffolie, gebildeter Beutel verstanden, der eingerichtet ist zur Aufnahme, zur Lagerung, zum Transport, und zum Prozessieren eines flüssigen Mediums, insbesondere eines flüssigen biologischen Mediums. Bekannte Folienbeutel dieser Art sind beispielsweise Blutbeutel oder Infusionsbeutel. Solche Folienbeutel werden aber auch verwendet um beispielsweise bei der Impfstoffherstellung Virussuspensionen aufzunehmen. Eine Vielzahl anderer Anwendungen sowie anderer flüssiger Medien in Zusammenhang mit solchen Folienbeuteln ist möglich. Der Folienbeutel weist insbesondere eine Basisfolie und eine Deckfolie auf, wobei zwischen der Basisfolie und der Deckfolie ein abgeschlossenes Kompartiment gebildet ist, in dem das flüssige Medium aufgenommen ist. Im Betrieb der hier vorgeschlagenen Vorrichtung liegt insbesondere die Basisfolie unmittelbar auf der Lauffläche auf, während die Deckfolie über die Basisfolie und das flüssige Medium gespannt wird.

Der Folienbeutel kann insbesondere als sogenannter Siegelrandbeutel ausgebildet sein. Dabei sind die Basisfolie und die Deckfolie separat voneinander bereitgestellt und über Schweißnähte, das heißt Siegelränder, derart miteinander verschweißt oder verklebt, dass sich zwischen der Basisfolie und der Deckfolie das abgeschlossene Kompartiment bildet. Alternativ ist der Folienbeutel als sogenannter Schlauchbeutel ausgebildet, wobei ein Folienschlauch abschnittsweise oder an Enden mit einer flachen Naht verschweißt oder verklebt ist, wodurch zwischen zwei Nähten das abgeschlossene Kompartiment gebildet ist. Durch die flachen Nähte wird der Schlauchbeutel insgesamt in eine abgeflachte Form gebracht, und es lassen sich auch wie bei dem Siegelrandbeutel ein oberer Folienabschnitt als Deckfolie und ein unterer Folienabschnitt als Basisfolie unterscheiden. Der Folienbeutel ist vorzugsweise aus zumindest einer flexiblen und in Grenzen elastischen Kunststofffolie gebildet, besonders bevorzugt aus PE oder PET/PE-Verbundmaterial, PP, oder auch PVC.

In der hier vorgeschlagenen Vorrichtung können auch Ketten hintereinander liegender getrennter Kompartimente als Folienbeutel-Kette verwendet werden. Dabei kann die hier vorgeschlagene Vorrichtung insbesondere auch Teil einer Automatenstraße oder Transportstraße sein, entlang der eine Mehrzahl von Folienbeutel, insbesondere eine Folienbeutel-Kette, gefördert wird.

Die Stauflächen sind insbesondere entlang der Transportrichtung voneinander durch den Stauabstand beabstandet angeordnet. Sie sind also insbesondere jeweils seitlich der Transportrichtung angeordnet, beidseitig eines zu transportierenden Folienbeutels.

Die Stauflächen sind vorzugsweise zumindest bereichsweise plan ausgebildet.

Das Bremselement ist insbesondere eingerichtet, um die Deckfolie des Folienbeutels abzubremsen.

Das Spannelement ist insbesondere eingerichtet, um die Deckfolie des Folienbeutels zu spannen.

Das Spannelement ist bevorzugt in Abhängigkeit von einer vorbestimmten, vorzugsweise parametrierbaren Schichtdicke des Flüssigkeitsfilms in dem Folienbeutel ansteuerbar.

Die Vorrichtung weist vorzugsweise als den Bandantrieb für das erste Transportband einen ersten Bandantrieb auf, der mit dem ersten Transportband antriebswirkverbunden und eingerichtet ist, um das erste Transportband anzutreiben. Alternativ ist es möglich, dass das erste Transportband dazu eingerichtet ist, mit einem separat von der Vorrichtung vorgesehenen Antriebsmittel oder Bandantrieb zusammenzuwirken, um durch dieses Antriebsmittel oder diesen Bandantrieb angetrieben zu werden.

Die Vorrichtung weist vorzugsweise eine Steuereinrichtung auf, die eingerichtet ist, um das Spannelement anzusteuern. In bevorzugter Ausgestaltung ist die Steuereinrichtung außerdem eingerichtet, um den ersten Bandantrieb oder den separaten Bandantrieb für das erste Transportband anzusteuern.

Vorzugsweise ist die erste Transportfläche vollständig durch das erste Transportband gebildet.

Die Lauffläche ist bevorzugt bereichsweise durch das erste Transportband gebildet. Dies stellt eine besonders einfache und integrierte Ausgestaltung der Vorrichtung dar, insbesondere da der Folienbeutel mittels des ersten Transportbands sowohl im Bereich der Stauflächen als auch im Bereich der Lauffläche transportiert werden kann.

Insbesondere ist die Lauffläche bevorzugt an einer Zylinderwalze ausgebildet, über welche das erste Transportband im Betrieb der Vorrichtung abläuft. Bevorzugt ist das erste Transportband zwischen zwei ersten Zylinderwalzen gespannt und läuft im Betrieb der Vorrichtung auf den beiden ersten Zylinderwalzen als Endlosband ab. Die Lauffläche ist dabei an einer ersten ersten Zylinderwalze der beiden ersten Zylinderwalzen angeordnet.

Das erste Transportband ist vorzugsweise aus Stahl, insbesondere als Stahlband gebildet oder besteht aus Stahl. Alternativ ist es möglich, dass das erste Transportband aus einem Kunststoff, insbesondere aus Fluorkautschuk (FKM), gebildet ist oder aus einem Kunststoff, insbesondere aus Fluorkautschuk (FKM), besteht.

Die Stauflächen sind vorzugsweise zumindest im Wesentlichen vertikal, vorzugsweise vertikal orientiert. Bevorzug läuft das erste Transportband im Betrieb der Vorrichtung im Bereich der ersten Staufläche vertikal aufwärts, der Folienbeutel wird also im Betrieb der Vorrichtung auch im Bereich der Stauflächen aufwärts transportiert. Die Lauffläche ist vorzugsweise an einem oberen Ende der Vorrichtung angeordnet.

Die Funktionsweise der Vorrichtung beruht - ohne an die Theorie gebunden sein zu wollen - zumindest ungefähr auf folgenden Überlegungen: Wird der Folienbeutel über der konvexen Lauffläche gespannt, ist die Deckfolie aufgrund des größeren Abstands zum Krümmungszentrum der konvexen Lauffläche entlang einer größeren Umfangsstrecke angeordnet als die unmittelbar auf der Lauffläche aufliegende Basisfolie. Die flexible und in Grenzen dehnbare Deckfolie wird dadurch im Bereich der Lauffläche straff gespannt, und es wird eine kontrollierte Engstelle lokal in dem Folienbeutel erzeugt, die dort die gewünschte Ausbildung des gleichmäßigen Flüssigkeitsfilms ermöglicht. Die Ausbildung des Flüssigkeitsfilms beim Transport des Folienbeutels über die Lauffläche erfolgt dadurch, dass in einer ersten Phase das flüssige Medium in dem noch vor dem Bremselement angeordneten Teil-Kompartiment des Folienbeutels aufgrund der durch die Bremswirkung des Bremselements und dem Aufspannen des Folienbeutels über der konvexen Laufstelle gebildeten Engstelle zurückgehalten wird, wobei es deswegen gegenüber dem Folienbeutel selbst zunächst nicht oder nur in geringerem Maß weitertransportiert wird. Da aber ein Aufblähen des zwischen den Stauflächen angeordneten Teil-Kompartiments durch die Volumenrestriktion in dem durch den Stauabstand gebildeten Stauraum verhindert wird, kommt es dort zu einer lokalen Druckerhöhung, und es wird in einer zweiten Phase das angestaute flüssige Medium innerhalb des Kompartiments aktiv in Transportrichtung des Folienbeutels gepumpt, wobei es aufgrund der Ausgestaltung der hier vorgeschlagenen Vorrichtung kontrolliert und gleichmäßig zwischen den an der konvexen Lauffläche aufgespannten Folien, das heißt der Basisfolie und der Deckfolie, hindurchströmt, dabei den Folienbeutel überholt und in dem hinter dem Spannelement angeordneten Teil-Kompartiment des Folienbeutels, das sich frei ausdehnen kann, aufgenommen wird. Somit liegt an der konvexen Lauffläche zwischen dem Bremselement und dem Spannelement ein Teil-Kompartiment des Folienbeutels vor, in dem das enthaltene flüssige Medium als dünner Flüssigkeitsfilm vorliegt, mit einem für eine dosiskontrollierte Exposition mit physikalischer Strahlung günstigen Oberflächen/Volumen-Verhältnis.

Der Flüssigkeitsfilm lässt sich dort bevorzugt mit einer homogenen Dicke von 200 µm oder weniger erzeugen. Bevorzugt beträgt die Schichtdicke von mindestens 20 µm bis höchstens 200 µm.

Die hier vorgeschlagene Vorrichtung ermöglicht insbesondere eine Regelung der Schichtdicke unabhängig von der Transportgeschwindigkeit, indem der Transport und die Erzeugung der Beutelspannung des Folienbeutels voneinander entkoppelt werden.

Gemäß einer Weiterbildung der Erfindung ist vorgesehen, dass eine zweite Staufläche der beiden Stauflächen zumindest bereichsweise durch ein zweites Transportband gebildet ist. Vorteilhaft können so beide Stauflächen zumindest bereichsweise durch Transportbänder gebildet sein, sodass ein besonders gut definierter Transport des Folienbeutels durch die Transportbänder und insbesondere zwischen den Transportbändern möglich ist.

Vorzugsweise ist die zweite Transportfläche vollständig durch das zweite Transportband gebildet.

Das zweite Transportband ist vorzugsweise aus Stahl gebildet, vorzugsweise besteht das zweite Transportband aus Stahl. Insbesondere ist es bevorzugt als Stahlband ausgebildet. Alternativ ist es möglich, dass das zweite Transportband aus einem Kunststoff, insbesondere Fluorkautschuk (FKM), gebildet ist, oder aus einem Kunststoff, insbesondere aus Fluorkautschuk (FKM), besteht.

Gemäß einer Weiterbildung der Erfindung ist vorgesehen, dass das erste Transportband auf einer ersten Stauplatte relativ zu der ersten Stauplatte verlagerbar angeordnet ist. Dies stellt vorteilhaft eine zumindest teilweise geführte und gestützte Verlagerung für das erste Transportband bereit. Außerdem nimmt die erste Stauplatte vorteilhaft einen Anpressdruck des Folienbeutels auf und stützt diesen ab.

Bevorzugt ist das zweite Transportband auf einer zweiten Stauplatte relativ zu der zweiten Stauplatte verlagerbar angeordnet. Auf diese Weise wird vorteilhaft das zweite Transportband zumindest bereichsweise geführt und gestützt, wobei der Anpressdruck des Folienbeutels auch durch die zweite Stauplatte aufgenommen und abgestützt werden kann.

Besonders bevorzugt ist das erste Transportband auf der ersten Stauplatte relativ zu dieser verlagerbar angeordnet, wobei das zweite Transportband auf der zweiten Stauplatte relativ zu dieser verlagerbar angeordnet ist. Dies ermöglicht eine besonders stabile Führung und Abstützung des Folienbeutels sowie der Transportbänder beim Transport des Folienbeutels. Der Folienbeutel kann dabei insbesondere zwischen den Stauplatten angeordnet sein und dort durch die Transportbänder transportiert werden.

Gemäß einer Weiterbildung der Erfindung ist vorgesehen, dass das erste Transportband und das zweite Transportband miteinander mechanisch bezüglich ihrer Laufgeschwindigkeit gekoppelt sind. Dies stellt eine besonders einfache Ausgestaltung der Vorrichtung dar, bei welcher insbesondere das zweite Transportband durch denselben Bandantrieb angetrieben werden kann wie das erste Transportband. Zugleich werden unerwünschte Laufgeschwindigkeitsunterschiede zwischen den Transportbändern vorteilhaft vermieden.

Alternativ ist bevorzugt vorgesehen, dass dem ersten Transportband und dem zweiten Transportband jeweils ein unabhängig ansteuerbarer Bandantrieb zugeordnet ist. Dies bedeutet insbesondere, dass der erste Bandantrieb für das erste Transportband unabhängig von einem zweiten Bandantrieb für das zweite Transportband ansteuerbar ist - und umgekehrt. Dies ermöglicht eine besonders flexible Ansteuerung der Bandantriebe und eine besonders flexible Wahl der verschiedenen Laufgeschwindigkeiten, insbesondere um Transportparameter für den Folienbeutel zusätzlich zu beeinflussen. Vorzugsweise sind dabei die Laufgeschwindigkeiten für das erste Transportband und das zweite Transportband - bevorzugt elektronisch - einstellbar. Insbesondere ist die Steuereinrichtung bevorzugt mit dem ersten Bandantrieb und mit dem zweiten Bandantrieb wirkverbunden und eingerichtet, um die Laufgeschwindigkeiten - insbesondere unabhängig voneinander - vorzugeben. Vorteilhaft wird es so möglich, die beiden Förderbänder verschieden schnell oder sogar - zumindest zeitweise - in verschiedene Richtungen zu bewegen, um so Transportparameter für den Folienbeutel bedarfsgerecht und besonders flexibel zu beeinflussen.

Es ist möglich, dass der Stauabstand entlang der Transportrichtung konstant ist, oder dass sich der Stauabstand alternativ kontinuierlich in Transportrichtung verjüngt. Insbesondere ist es möglich, dass die erste Staufläche und die zweite Staufläche plan, vorzugsweise planparallel zueinander, ausgebildet sind. Der Stauabstand kann beispielsweise ungefähr 1 mm betragen, bevorzugt beträgt er höchstens 1 mm oder ist kleiner als 1 mm.

Es ist in bevorzugter Ausgestaltung auch möglich, dass der Stauabstand entlang der Transportrichtung eine diskrete Änderung, insbesondere in Transportrichtung eine diskrete Verjüngung aufweist. Bevorzugt weist zumindest eine der Stauflächen, ausgewählt aus der ersten Staufläche und der zweiten Staufläche, eine Stufe auf, wobei sich der Stauabstand im Bereich der Stufe ändert, insbesondere verjüngt, insbesondere von einem ersten konstanten Abstandswert vor der Stufe - beispielsweise 1 mm - zu einem zweiten, kleineren konstanten Abstandswert hinter der Stufe - beispielsweise 0,5 mm. Der zweite Abstandswert beträgt bevorzugt ungefähr die Hälfte des ersten Abstandswerts, vorzugsweise die Hälfte des ersten Abstandswerts. Die Änderung, Verjüngung oder Stufe ist bevorzugt in einem Bereich vor dem Bremselement angeordnet, insbesondere - in Transportrichtung gemessen - in einem Abstand zu dem Bremselement, der kleiner ist als die Hälfte der in Transportrichtung gemessenen Länge einer der Stauflächen, insbesondere der die Stufe aufweisenden Staufläche, vorzugsweise kleiner als ein Drittel der Länge, insbesondere kleiner als ein Viertel der Länge.

Gemäß einer Weiterbildung der Erfindung ist vorgesehen, dass zumindest eine der Stauflächen derart relativ zu der anderen Stauflächen der beiden Stauflächen verlagerbar ist, dass der Stauabstand einstellbar ist. Dies ermöglicht vorteilhaft eine Einstellung des Stauabstands insbesondere als weiteren Transportparameter mit Blick auf den Transport des Folienbeutels. Insbesondere kann so eine Reibkraft zwischen dem Folienbeutel und den Stauflächen variiert werden - vorzugsweise auch dynamisch während des Transports des Folienbeutels durch die Vorrichtung.

Gemäß einer bevorzugten Ausgestaltung ist der Stauabstand entlang der Transportrichtung konstant einstellbar. In diesem Fall sind die Stauflächen zumindest im Wesentlichen parallel zueinander, vorzugsweise parallel zueinander, angeordnet.

Alternativ oder zusätzlich ist bevorzugt vorgesehen, dass der Stauabstand sich entlang der Transportrichtung verjüngend eingestellt werden kann. Die Stauflächen sind dann zumindest geringfügig schräg zueinander orientiert, quasi in Form eines Keils angeordnet, wobei der Stauabstand entlang der Transportrichtung gesehen zwischen den Stauflächen kleiner wird. Somit wird insbesondere die Reibung zwischen dem Folienbeutel und den Stauflächen zu dem Bremselement hin größer. Durch Einstellen der Verjüngung des Stauabstands kann ein weiterer Transportparameter vorteilhaft variiert werden.

Gemäß einer Weiterbildung der Erfindung ist vorgesehen, dass das Bremselement als Bremsrolle ausgebildet ist. Bevorzugt weist die Bremsrolle wenigstens ein haftreibungserhöhendes Element auf, besonders bevorzugt eine Mehrzahl von auf einen Walzengrundkörper der Bremsrolle aufgezogenen, zueinander in Axialrichtung der Bremsrolle beabstandet angeordneten Reibringen. Die Reibringe sind in bevorzugter Ausgestaltung als O-Ringe ausgebildet.

Vorzugsweise ist die Bremsrolle mit dem ersten Transportband geschwindigkeitsgekoppelt. Dies stellt eine besonders einfache Ausgestaltung der Bremsrolle als passiv gebremstes Bremselement dar, wobei es weder separater Bremseinrichtungen noch eines eigenen Antriebs für die Bremsrolle bedarf. Insbesondere ist die Bremsrolle bevorzugt dadurch mit dem ersten Transportband geschwindigkeitsgekoppelt, dass sie mit dem zumindest einen haftreibungserhöhenden Element an dem ersten Transportband anliegt und dadurch mit dem ersten Transportband reibschlüssig gekoppelt ist. Sie wird somit insbesondere durch das erste Transportband synchron mitbewegt und kann vorzugsweise zumindest durch die im Betrieb der Vorrichtung im normalen Funktionsfall auftretenden Kräfte oder Momente nicht relativ zu dem ersten Transportband beschleunigt werden.

Alternativ ist bevorzugt vorgesehen, dass die Bremsrolle unabhängig von dem ersten Transportband angetrieben ist. Dies ermöglicht eine besonders flexible Einstellung der Transportparameter, sowie eine zusätzliche Beeinflussung der Beutelspannung. Bei dieser Ausgestaltung kann sich die Bremsrolle insbesondere mit von der Laufgeschwindigkeit des ersten Transportbands unabhängiger Drehzahl drehen.

Alternativ ist es bevorzugt möglich, dass die Bremsrolle aktiv oder passiv gebremst ist. Eine aktive Bremsung kann beispielsweise bewirkt werden durch einen ansteuerbaren Bremsmechanismus, beispielsweise Bremsbacken, oder durch eine mit der Bremsrolle wirkverbundene elektrische Maschine, die in Bremsrichtung angesteuert, beispielsweise als Generator betrieben wird. Eine passive Bremsung kann beispielsweise durch eine geeignet eingestellte Fliehkraftbremse bereitgestellt werden.

Gemäß einer Weiterbildung der Erfindung ist vorgesehen, dass das Spannelement als Spannrolle ausgebildet ist. Dies stellt ebenfalls eine einfache sowie zuverlässige und funktionale Ausgestaltung der Vorrichtung dar. Die mit dem Spannelement wirkverbundene Steuereinrichtung ist insbesondere eingerichtet, um das Spannelement in Abhängigkeit von der bestimmten, vorzugsweise parametrierbaren Schichtdicke des zu erzeugenden Flüssigkeitsfilms drehmomentgesteuert, vorzugsweise drehmomentgeregelt, oder drehzahlgesteuert, vorzugsweise drehzahlgeregelt, anzusteuern. Insbesondere wenn das Spannelement drehmomentgesteuert oder drehmomentgeregelt angesteuert wird, erfolgt dies bevorzugt so, dass kein Schlupf zwischen dem Spannelement, insbesondere der Spannrolle, und der Deckfolie des Folienbeutels auftritt. Die Deckfolie wird somit vorteilhaft - insbesondere haftreibungsvermittelt - zeitlich konstant mit einer bestimmten, sich aus dem Drehmoment der Spannrolle ergebenden Spannkraft beaufschlagt, woraus die Beutelspannung resultiert.

Eine drehzahlgesteuerte, vorzugsweise drehzahlgeregelte Ansteuerung des Spannelements ist demgegenüber eine einfachere Ausgestaltung, die gleichwohl eine gute Definition der Beutelspannung ermöglicht. Dabei wird allerdings gegebenenfalls ein Schlupf zwischen der Deckfolie und der Spannrolle in Kauf genommen. Die in die Deckfolie eingeleitete Spannkraft kann sich dabei insbesondere auch aus einer Gleitreibung zwischen der Spannrolle und der Deckfolie ergeben.

Gemäß einer Weiterbildung der Erfindung ist vorgesehen, dass die Vorrichtung eine Temperiereinrichtung aufweist, die eingerichtet ist, um die Lauffläche zu temperieren. Auf diese Weise kann vorteilhaft die Temperatur des Flüssigkeitsfilms eingestellt werden, insbesondere in einem für die Exposition mit physikalischer Strahlung beziehungsweise die angestrebte Wirkung günstigen Bereich.

Die Temperiereinrichtung ist bevorzugt eingerichtet, um die Lauffläche zu erwärmen.

Alternativ oder zusätzlich weist die Vorrichtung bevorzugt eine Kühleinrichtung auf, die eingerichtet ist, um das Spannelement zu kühlen. Mit dem Spannelement kann dabei bevorzugt zugleich der Folienbeutel und damit das in dem Folienbeutel angeordnete flüssige Medium gekühlt, insbesondere nach einem möglichen Erwärmen im Bereich der Lauffläche wieder abgekühlt werden. Dies ermöglicht vorteilhaft, dass eine gegebenenfalls zur Behandlung vorgesehene Temperaturerhöhung für das flüssige Medium nur vergleichsweise kurzzeitig erfolgt, sodass insbesondere eine Dauerhaltbarkeit des flüssigen Mediums nicht gefährdet ist.

Gemäß einer Weiterbildung der Erfindung ist vorgesehen, dass die Vorrichtung eine Messeinrichtung zur Ermittlung einer Schichtdicke des Flüssigkeitsfilms aufweist. Auf diese Weise kann vorteilhaft die Schichtdicke erfasst und insbesondere - vorzugsweise durch Ansteuern des ansteuerbaren Spannelements und/oder zumindest des ersten Transportbands - eingeregelt werden.

"Ermittlung der Schichtdicke" bedeutet dabei nicht notwendig, dass die Schichtdicke explizit bestimmt wird. Es genügt vielmehr zum Zweck eines reproduzierbaren Betriebs der Vorrichtung unter Einhaltung einer zeitlich konstanten Schichtdicke sowie insbesondere einer kontrollierten Exposition des flüssigen Mediums mit physikalischer Strahlung, wenn indirekt eine physikalische Größe erfasst wird, die als Maß für die Schichtdicke dienen kann oder zumindest eindeutig mit der Schichtdicke zusammenhängt, wobei diese physikalische Größe derart eingeregelt wird, dass die Schichtdicke zeitlich konstant ist. Selbstverständlich ist es aber auch möglich, dass die Schichtdicke explizit erfasst und vorzugsweise eingeregelt wird.

Die Messeinrichtung ist bevorzugt eingerichtet, um die Schichtdicke mittels Druckmessung, optisch, elektrisch oder elektronisch, und/oder durch Erfassen einer Strömungsgeschwindigkeit des flüssigen Mediums in dem Folienbeutel zu ermitteln.

Für eine Ermittlung mittels Druckmessung ist vorzugsweise ein Drucksensor vorgesehen, der in Transportrichtung vor dem Bremselement angeordnet ist. Über den Drucksensor wird bevorzugt ein Druck als Maß für einen Strömungswiderstand für das flüssige Medium erfasst, der von der Schichtdicke auf der Lauffläche abhängig ist. Der Druck wird bevorzugt kontinuierlich oder in diskreten Zeitabständen erfasst. Insbesondere wird bevorzugt eine relative zeitliche Änderung des mit dem Drucksensor erfassten Drucks ausgewertet. Mittels eines Modells kann dann bevorzugt auf die Schichtdicke zurückgerechnet werden, und/oder der Druck kann in einem vorgegebenen Druckbereich konstant eingeregelt werden, um die Schichtdicke konstant zu halten.

Eine optische Ermittlung der Schichtdicke ist vorzugsweise ausgewählt aus einer Gruppe, bestehend aus: Einem bildgebenden Verfahren, dem Erfassen einer Abschattung, dem Erfassen einer Intensität, insbesondere einer Farbe oder Helligkeit des Flüssigkeitsfilms auf der Lauffläche, und einer Interferenzmessung.

Ein bildgebendes Verfahren sowie ein Erfassen einer Abschattung, insbesondere das Erfassen der Abschattung einer Lichtquelle durch den Flüssigkeitsfilm, ermöglicht jeweils eine direkte Messung der Schichthöhe.

Die Messung einer Intensität, insbesondere einer Farbintensität und/oder einer Helligkeit des Flüssigkeitsfilms ermöglicht ebenfalls eine Bestimmung der Schichtdicke, insbesondere wenn das flüssige Medium lumineszent, fluoreszent ausgebildet oder anderweitig gefärbt ist.

Schließlich ermöglicht auch eine Interferenzmessung eine direkte Bestimmung der Schichtdicke, insbesondere indem die Vorrichtung ein spaltbildendes Element aufweist, das gegenüber der Lauffläche derart angeordnet ist, dass ein optischer Spalt einerseits durch die Deckfolie im höchsten Punkt der konvexen Lauffläche und damit zugleich im Maximum des Flüssigkeitsfilms und andererseits durch das spaltbildende Element begrenzt ist. Somit hängt ein in Transmission durch den derart gebildeten Spalt entstehendes Interferenzmuster unmittelbar von der Schichtdicke ab.

Eine elektrische oder elektronische Messung schließt insbesondere eine kapazitive Messung und/oder eine Messung mittels Hochfrequenz-Verstimmung der Schichtdicke ein.

Da der Flüssigkeitsfilm insbesondere dynamisch durch das in dem Folienbeutel strömende und insbesondere im Bereich der Lauffläche den Folienbeutel überholende flüssige Medium gebildet wird, hängt die Schichtdicke direkt mit der Strömungsgeschwindigkeit des flüssigen Mediums in dem Folienbeutel zusammen. Somit kann die Schichtdicke auch ermittelt werden, indem die Strömungsgeschwindigkeit des flüssigen Mediums in dem Folienbeutel im Bereich der Lauffläche erfasst wird. Dies kann beispielsweise über ein PIV-Verfahren (Particle Image Velocimetry), ein Ultraschallverfahren, oder ein anderes kontaktloses Strömungsgeschwindigkeitsmessverfahren, erfasst werden. Vorzugsweise erfolgt eine anlauf- und ablaufseitige Messung der Strömungsgeschwindigkeit.

Die Aufgabe wird schließlich auch gelöst, indem eine Anordnung zur kontrollierten Exposition eines flüssigen Mediums in einem Folienbeutel mit physikalischer Strahlung geschaffen wird, wobei die Anordnung eine erfindungsgemäße Vorrichtung oder eine Vorrichtung nach einem der zuvor beschriebenen Ausführungsbeispiele aufweist. Die Anordnung weist außerdem eine Strahlungsquelle physikalischer Strahlung auf, die angeordnet und eingerichtet ist, um den Folienbeutel im Bereich der konvexen Lauffläche zu bestrahlen. In Zusammenhang mit der Anordnung ergeben sich insbesondere die Vorteile, die bereits in Zusammenhang mit der Vorrichtung erläutert wurden. Insbesondere erfolgt die Bestrahlung dort, wo der Flüssigkeitsfilm mit - durch die hier vorgeschlagene Vorrichtung - gut definierter und zeitlich konstanter Schichtdicke erzeugt ist, insbesondere im Maximum der konvexen Lauffläche. Somit kann die Exposition des flüssigen Mediums mit der physikalischen Strahlung insbesondere dosiskontrolliert erfolgen.

Unter physikalischer Strahlung wird allgemein ein physikalisches Strahlungsphänomen, sei es Teilchenstrahlung oder Wellenstrahlung, verstanden. Bevorzugt ist die Strahlungsquelle eingerichtet zur Emission von ionisierender Strahlung. Vorzugsweise ist die Strahlungsquelle eingerichtet zur Emission von elektromagnetischer Strahlung oder Teilchenstrahlung, insbesondere von Betastrahlung, harter oder weicher Röntgenstrahlung, ultravioletter Strahlung, sichtbarer elektromagnetischer Strahlung (Licht), Infrarot- oder Wärmestrahlung, Terahertzstrahlung, Mikrowellenstrahlung, Atomionenstrahlung, Protonenstrahlung, Positronenstrahlung, oder einer anderen Art von Strahlung.

Die Aufgabe wird schließlich auch gelöst, indem ein Verfahren zum Erzeugen eines Flüssigkeitsfilms eines flüssigen Mediums in einem Folienbeutel geschaffen wird, wobei ein Folienbeutel in Transportrichtung durch eine erfindungsgemäße Vorrichtung oder eine Vorrichtung nach einem der zuvor beschriebenen Ausführungsbeispiele transportiert wird, und wobei ein gleichmäßiger, vorzugsweise eine zeitlich konstante Schichtdicke aufweisender Flüssigkeitsfilm erzeugt wird, indem zumindest ein Bandantrieb für das erste Transportband einerseits, und das Spannelement andererseits, unabhängig voneinander angesteuert werden. In Zusammenhang mit dem Verfahren ergeben sich insbesondere die bereits in Zusammenhang mit der Vorrichtung und der Anordnung beschriebenen Vorteile.

Dass der Bandantrieb einerseits und das Spannelement andererseits unabhängig voneinander angesteuert werden, bedeutet insbesondere, dass keine direkte Abhängigkeit zwischen der Ansteuerung des Bandantriebs für das erste Transportband und der Ansteuerung des Spannelements gegeben ist. Eine indirekte oder mittelbare Abhängigkeit ist dabei nicht ausgeschlossen, insbesondere nicht dergestalt, dass die Regelung der Schichtdicke als übergeordnete Größe eine Anpassung sowohl der Ansteuerung des Bandantriebs als auch der Ansteuerung des Spannelements und gegebenenfalls sogar eine Abstimmung der Ansteuerung des Bandantriebs und der Ansteuerung des Spannelements aufeinander verlangen kann. Es erfolgt aber insbesondere keine direkte mechanische, elektronische oder anderweitige logische Kopplung zwischen dem Bandantrieb und der Ansteuerung des Spannelements in dem Sinn, dass eine Änderung der Ansteuerung des einen Elements unmittelbar eine Änderung der Ansteuerung des anderen Elements zur Folge hat.

Die Erfindung wird im Folgenden anhand der Zeichnung näher erläutert. Dabei zeigen:
- Figur 1: eine Darstellung eines Ausführungsbeispiels einer Anordnung mit einem ersten Ausführungsbeispiel einer Vorrichtung zur Erzeugung eines Flüssigkeitsfilms eines flüssigen Mediums in einem Folienbeutel,
- Figur 2: eine Seitenansicht der Vorrichtung gemäß Figur 1 in zwei Funktionsstellungen,
- Figur 3: eine Draufsicht auf die Vorrichtung gemäß Figur 1,
- Figur 4: eine erste Detailansicht der Vorrichtung gemäß Figur 1,
- Figur 5: eine schematische Detaildarstellung eines zweiten Ausführungsbeispiels der Vorrichtung, und
- Figur 6: eine schematische Detaildarstellung eines dritten Ausführungsbeispiels der Vorrichtung.

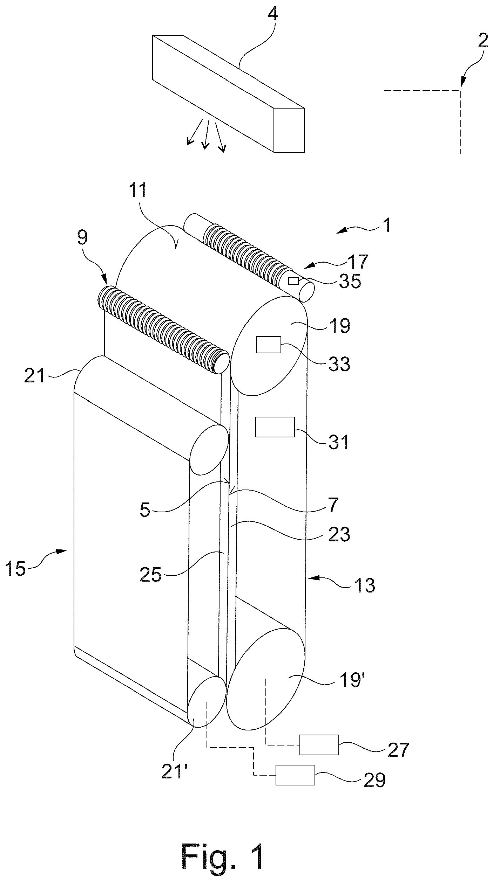

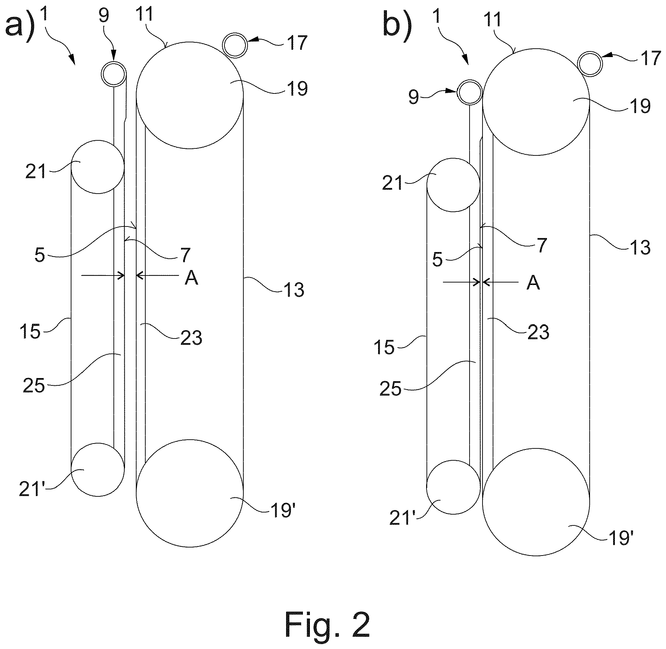

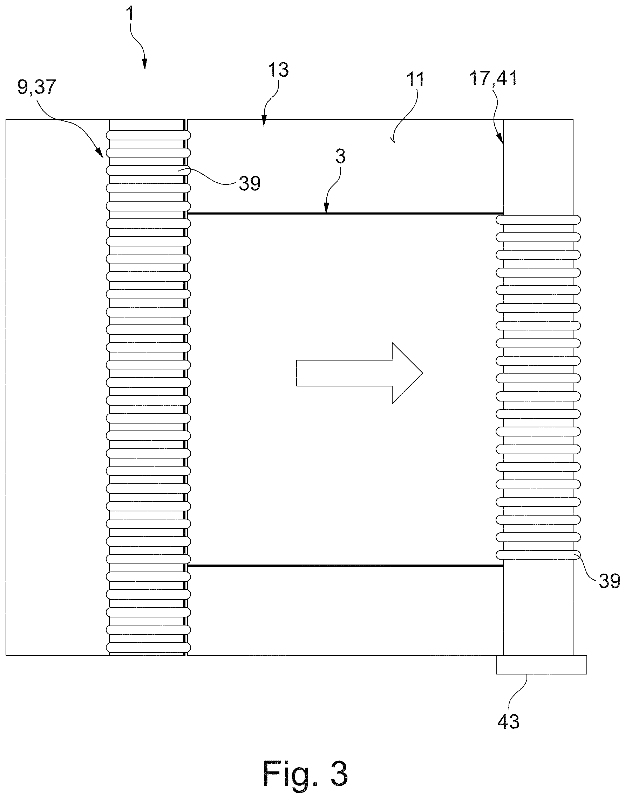

**Fig. 1** zeigt insbesondere eine schematische Darstellung eines ersten Ausführungsbeispiels einer Vorrichtung 1 zur Erzeugung eines Flüssigkeitsfilms eines flüssigen Mediums in einem schematisch in Figur 3 dargestellten Folienbeutel 3. Die Vorrichtung 1 weist zwei voneinander durch einen in Figur 2 dargestellten Stauabstand A beabstandete Stauflächen auf, nämlich eine erste Staufläche 5 und eine zweite Staufläche 7, zwischen denen der Folienbeutel 3 angeordnet und entlang einer Transportrichtung, in Figur 1 vertikal nach oben, transportiert werden kann. In Transportrichtung hinter den Stauflächen 5, 7 ist ein Bremselement 9 angeordnet, das eingerichtet ist, um den Folienbeutel 3 abzubremsen. Die Vorrichtung 1 weist außerdem eine in Transportrichtung konvex gekrümmte Lauffläche 11 auf, die derart eingerichtet und angeordnet ist, dass der durch das Bremselement 9 gebremste Folienbeutel 3 auf der Lauffläche 11 hinter dem Bremselement 9 aufgespannt werden kann. Im Bereich der Lauffläche 11 wird auch die Transportrichtung entsprechend der Krümmung der Lauffläche 11 umgelenkt; die Transportrichtung folgt also insbesondere der Krümmung der Lauffläche 11, wie auch der transportierte Folienbeutel 3.

Die erste Staufläche 5 ist zumindest bereichsweise, vorzugsweise vollständig, durch ein angetriebenes erstes Transportband 13 gebildet, das eingerichtet ist, um den Folienbeutel 3 in Transportrichtung zu fördern.

Bei dem hier dargestellten Ausführungsbeispiel ist in bevorzugter Weise auch die zweite Staufläche 7 zumindest bereichsweise, vorzugsweise vollständig, durch ein bevorzugt angetriebenes zweites Transportband 15 gebildet.

Die Vorrichtung 1 weist in Transportrichtung hinter dem Bremselement 9 ein ansteuerbares Spannelement 17 auf, das eingerichtet ist, um den Folienbeutel 3 auf der Lauffläche 11 zwischen dem Bremselement 9 und dem Spannelement 17 aufzuspannen. Das erste Transportband 13 oder ein Bandantrieb für das erste Transportband 13 ist unabhängig von dem ansteuerbaren Spannelement 17 ansteuerbar.

Mit dem angetriebenen ersten Transportband 13 und dem ansteuerbaren Spannelement 17 ist es insbesondere vorteilhaft möglich, die Beutelspannung des Folienbeutels 3 im Bereich der Lauffläche 11 einerseits und die Transportgeschwindigkeit des Folienbeutels 3 andererseits unabhängig voneinander einzustellen, insbesondere zu regeln. Insbesondere auf diese Weise kann im Bereich der Lauffläche 11 eine homogene und zeitlich konstante Schichtdicke des Flüssigkeitsfilms in dem Folienbeutel 3 gewährleistet werden. Dies ist insbesondere vorteilhaft mit Blick auf eine mithilfe der Vorrichtung 1 durchzuführende dosiskontrollierte Exposition des flüssigen Mediums mit physikalischer Strahlung.

Die Lauffläche 11 ist bevorzugt zumindest bereichsweise, vorzugsweise vollständig, durch das erste Transportband 13 gebildet. Insbesondere ist die Lauffläche 11 hier an einer ersten ersten Zylinderwalze 19 angeordnet, wobei das erste Transportband 13 auf zwei ersten Zylinderwalzen 19, 19', hier nämlich der ersten ersten Zylinderwalze 19 und einer zweiten ersten Zylinderwalze 19`, abläuft und vorzugsweise durch die ersten Zylinderwalzen 19, 19` gespannt ist.

Das zweite Transportband 15 läuft vorzugsweise auf zwei zweiten Zylinderwalzen 21, 21' ab und ist bevorzugt zwischen den zweiten Zylinderwalzen 21, 21' gespannt. Insbesondere sind so das erste Transportband 13 und das zweite Transportband 15 bevorzugt als Endlosbänder ausgebildet.

Das erste Transportband 13 ist hier auf einer ersten Stauplatte 23 angeordnet und relativ zu der ersten Stauplatte 23 verlagerbar. Das zweite Transportband 15 ist auf einer zweiten Stauplatte 25 angeordnet und relativ zu der zweiten Stauplatte 25 verlagerbar. Der Folienbeutel 3 ist dabei zwischen den Stauplatten 23, 25 anordenbar und wird dort durch die Transportbänder 13, 15 transportiert. Der Stauabstand A wird vorzugsweise insbesondere durch einen Abstand der Stauplatten 23, 25 voneinander definiert.

Gemäß einer bevorzugten Ausgestaltung sind das erste Transportband 13 und das zweite Transportband 15 miteinander mechanisch bezüglich ihrer Laufgeschwindigkeit gekoppelt. Hierzu kann beispielsweise vorteilhaft eine der ersten Zylinderwalzen 19, 19` mit einer der zweiten Zylinderwalzen 21, 21' mechanisch, beispielsweise über einen Riementrieb oder Zahnradtrieb oder in anderer geeigneter Weise, wirkverbunden sein, um eine synchrone Bewegung der Transportbänder 13, 15 durch einen gemeinsamen Bandantrieb zu bewirken.

Alternativ ist es aber möglich, dass dem ersten Transportband 13 und dem zweiten Transportband 15 jeweils ein unabhängig ansteuerbarer Bandantrieb zugeordnet ist, hier beispielsweise - lediglich schematisch dargestellt - dem ersten Transportband 13 ein erster Bandantrieb 27 und dem zweiten Transportband 15 ein zweiter Bandantrieb 29, wobei bevorzugt die Laufgeschwindigkeiten für das erste Transportband 13 und das zweite Transportband 15 - vorzugsweise elektronisch - einstellbar sind. Die Bandantriebe 27, 29 können auch anderen Zylinderwalzen 19, 19`, 21, 21' als hier dargestellt, insbesondere in bevorzugter Ausgestaltung den in der Figur oberen Zylinderwalzen 19, 21, zugeordnet sein.

Insbesondere weist die Vorrichtung 1 bevorzugt eine Steuereinrichtung 31 auf, die in hier nicht ausdrücklich dargestellter Weise mit dem wenigstens einen Bandantrieb, insbesondere mit dem ersten Bandantrieb 27 und mit dem zweiten Bandantrieb 29, wirkverbunden ist, um den wenigstens einen Bandantrieb, insbesondere beide Bandantriebe 27, 29, anzusteuern.

Unabhängig ansteuerbare Bandantriebe 27, 29 für das erste Transportband 13 und das zweite Transportband 15 ermöglichen eine weitergehende Variation von Transportparametern für den Folienbeutel 3, wobei es insbesondere möglich ist, die Transportbänder 13, 15 verschieden schnell oder sogar in verschiedene Richtungen zu bewegen.

Die Vorrichtung 1 weist bevorzugt eine Temperiereinrichtung 33 auf, die eingerichtet ist, um die Lauffläche 11 zu temperieren, insbesondere zu erwärmen. Die Temperiereinrichtung 33 ist vorzugsweise mit der Steuereinrichtung 31 wirkverbunden und durch diese ansteuerbar.

Alternativ oder zusätzlich weist die Vorrichtung 1 bevorzugt eine Kühleinrichtung 35 auf, die eingerichtet ist, um das Spannelement 17 zu kühlen. Auch die Kühleinrichtung 35 ist in bevorzugter Ausgestaltung mit der Steuereinrichtung 31 wirkverbunden und durch diese ansteuerbar.

Die Vorrichtung 1 ist bevorzugt Teil einer Anordnung 2 zur kontrollierten Exposition des flüssigen Mediums in dem Folienbeutel 3 mit physikalischer Strahlung. Dabei umfasst die Anordnung 2 neben der Vorrichtung 1 eine Strahlungsquelle 4, die eingerichtet ist zur Emission von physikalischer Strahlung. Die Strahlungsquelle 4 ist derart angeordnet, dass durch sie der Folienbeutel 3 im Bereich der konvexen Lauffläche 11 bestrahlt werden kann. Die Strahlungsquelle 4 ist insbesondere eingerichtet zur Emission von Teilchenstrahlung und/oder Wellenstrahlung, insbesondere von ionisierender Strahlung, insbesondere Betastrahlung, harter oder weicher Röntgenstrahlung, ultravioletter Strahlung, sichtbarem Licht, Infrarot- oder Wärmestrahlung, Terahertzstrahlung, Mikrowellenstrahlung, Atomionenstrahlung, Protonenstrahlung, Positronenstrahlung, oder einer anderen Form von physikalischer Strahlung.

**Fig. 2** zeigt eine Darstellung der Vorrichtung 1 gemäß Figur 1 in zwei verschiedenen Funktionsstellungen.

Gleiche und funktionsgleiche Elemente sind in allen Figuren mit gleichen Bezugszeichen versehen, sodass insoweit jeweils auf die vorangegangene Beschreibung verwiesen wird.

In Figur 2 ist bei a) die Vorrichtung 1 in einer ersten Funktionsstellung dargestellt, in welcher die Transportbänder 13, 15 sowie die Stauplatten 23, 25 vergleichsweise weit voneinander entfernt angeordnet sind, sodass der Stauabstand A vergleichsweise groß ist. In dieser ersten Funktionsstellung kann insbesondere der Folienbeutel 3 zwischen die Transportbänder 13, 15 eingelegt werden. Bei b) sind die Transportbänder 13, 15 sowie die Stauplatten 23, 25 in einer zweiten Funktionsstellung näher aneinander angeordnet, sodass der Stauabstand A geringer ausgebildet ist. Der Folienbeutel 3 wird insbesondere in dieser zweiten Funktionsstellung zwischen den Stauplatten 23, 25 und den Transportbändern 13, 15 gepresst, wodurch sich das flüssige Medium im Folienbeutel 3 gleichmäßig auf ein definiertes Volumen verteilt. Indem die Transportbänder 13, 15 geeignet angetrieben werden, kann nun der Folienbeutel 3 transportiert, insbesondere unter der Strahlungsquelle 4 hindurchgeführt werden.

Insgesamt ist bevorzugt zumindest eine der Stauflächen 5, 7, vorzugsweise die erste Staufläche 5, derart relativ zu der anderen Staufläche 7, 5 verlagerbar, dass der Stauabstand A einstellbar ist.

Dabei ist dieser vorzugsweise entlang der Transportrichtung konstant und/oder sich entlang der Transportrichtung verjüngend einstellbar.

In Figur 2 ist dabei lediglich eine konstante Einstellung des Stauabstands A dargestellt. Die Stauflächen 5, 7 sind insoweit parallel zueinander angeordnet. Kann zusätzlich noch ein Winkel zwischen den Stauflächen 5, 7 verändert werden, sodass sich der Stauabstand A entlang der Transportrichtung, in Figur 2 von vertikal unten nach vertikal oben, verjüngt, ist ein weiterer Freiheitsgrad zur Veränderung der Transportbedingungen für den Folienbeutel 3 bereitgestellt.

**Fig. 3** zeigt eine Draufsicht auf die Vorrichtung 1 gemäß den Figuren 1 und 2.

Das Bremselement 9 ist bevorzugt als Bremsrolle 37 ausgebildet. Die Bremsrolle 37 ist bei dem hier darstellten Ausführungsbeispiel in bevorzugter Ausgestaltung mit dem ersten Transportband 13 geschwindigkeitsgekoppelt. Dies ist bevorzugt dadurch erreicht, dass die Bremsrolle 37 reibschlüssig mit dem ersten Transportband 13 verbunden ist.

Konkret ist auf der Bremsrolle 37 eine Mehrzahl von in Axialrichtung der Bremsrolle 37 zueinander beabstandeten Reibringen 39 angeordnet, die nicht nur in einem mittleren Bereich der Bremsrolle 37 in Kontakt zu dem Folienbeutel 3 sind, sondern auch in seitlichen Randbereichen der Bremsrolle 37 in reibschlüssigem Kontakt mit dem ersten Transportband 13 stehen. Somit ist auf einfache Weise die Drehzahl der Bremsrolle 37 an die Laufgeschwindigkeit des ersten Transportbands 13 gekoppelt. Dies stellt insbesondere eine spezielle Ausgestaltung einer passiv gebremsten Bremsrolle 37 dar.

Es ist bei einer alternativen Ausgestaltung möglich, dass die Bremsrolle 37 unabhängig von dem ersten Transportband 13 angetrieben ist. Alternativ kann sie auch aktiv gebremst oder in anderer Ausgestaltung passiv gebremst ausgebildet sein, beispielsweise mittels einer Fliehkraftbremse.

Das Spannelement 17 ist bevorzugt als Spannrolle 41 ausgebildet. Vorzugsweise ist auch die Spannrolle 41 bereichsweise mit in Axialrichtung der Spannrolle 41 zueinander beabstandet angeordneten Reibringen 39 bestückt, allerdings bevorzugt lediglich in einem mittleren Bereich, wo die Reibringe 39 nur in reibschlüssigen Kontakt zu dem Folienbeutel 3 kommen; dagegen weist die Spannrolle 41 bevorzugt keine Reibringe 39 seitlich des Bereichs des Folienbeutels 3 auf, sodass die Spannrolle 41 nicht mit dem ersten Transportband 13 drehzahlgekoppelt ist.

Die Steuereinrichtung 31 ist bevorzugt mit dem Spannelement 17, insbesondere mit einem separaten Rollenantrieb 43 der Spannrolle 41, wirkverbunden und eingerichtet, um das Spannelement 17, hier die Spannrolle 41, in Abhängigkeit von einer bestimmten, bevorzugt parametrierbaren Schichtdicke des zu erzeugenden Flüssigkeitsfilms anzusteuern, insbesondere um den Rollenantrieb 43 entsprechend anzusteuern. Dabei erfolgt die Ansteuerung bevorzugt drehmomentgesteuert, vorzugsweise drehmomentgeregelt, oder drehzahlgesteuert, vorzugsweise drehzahlgeregelt.

Insbesondere wird durch den reibschlüssigen Kontakt zwischen der Spannrolle 41 und dem Folienbeutel 3 bevorzugt die Deckfolie des Folienbeutels 3 gespannt, sodass im Zusammenwirken mit der Bremsrolle 37, der Lauffläche 11 und den Transportbändern 13, 15 ein Flüssigkeitsfilm bestimmter, vorzugsweise parametrierbarer Schichtdicke in dem Folienbeutel 3 auf der Lauffläche 11 erzeugt werden kann.

Der Folienbeutel 3 wird somit auf der Oberseite des ersten Transportbands 13 zwischen der Bremsrolle 37 und der Spannrolle 41 eingespannt. Die Bremsrolle 37 ist reibschlüssig mit dem ersten Transportband 13 und mit dem Folienbeutel 3 gekoppelt. Die Spannrolle 41 liegt auf der Deckfolie des Folienbeutels 3 auf, sodass der Folienbeutel 3 zwischen der Bremsrolle 37 und der Spannrolle 41 eingespannt werden kann. Eine definierte Beutelspannung führt dabei zu einer homogenen, zeitlich konstanten Schichtdicke des flüssigen Mediums im Bereich der Lauffläche 11. Zum Einstellen der Schichtdicke ist dabei insbesondere das Drehmoment oder die Drehzahl der Spannrolle 41 maßgeblich, insbesondere abhängig davon, ob die Beutelspannung unter Beibehaltung der Haftreibung zwischen der Spannrolle 41 und der Deckfolie eingeregelt werden soll, also insbesondere unter Vermeidung eines Schlupfs zwischen Spannrolle 41 und Deckfolie, oder ob ein zeitweiser Schlupf zugelassen wird, wobei die Beutelspannung dann auch mittels Gleitreibung zwischen der Spannrolle 41 und der Deckfolie eingeregelt wird.

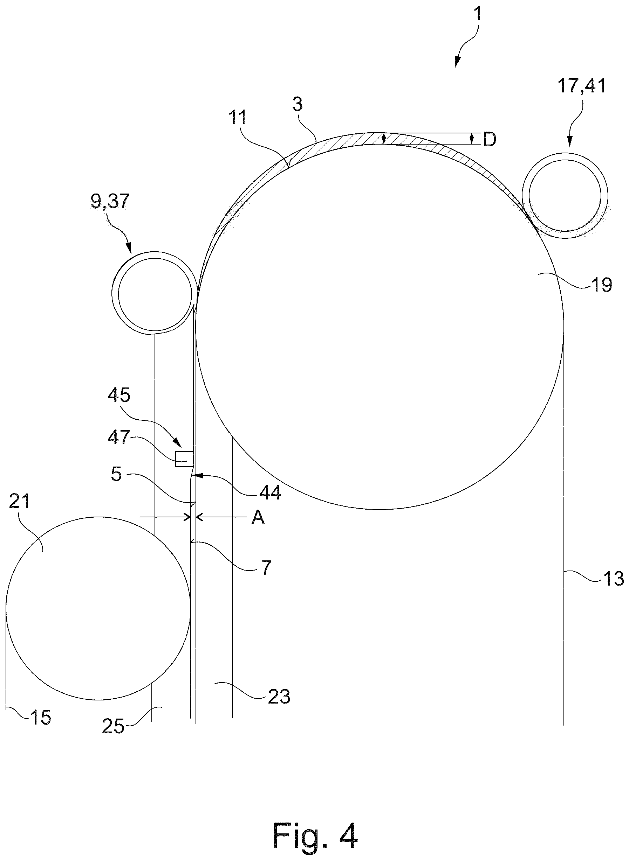

**Fig. 4** zeigt eine Detaildarstellung des Ausführungsbeispiels der Vorrichtung 1 gemäß den Figuren 1 bis 3 im Betrieb, wobei hier eine einzuregelnde, vorbestimmte Schichtdicke D des Flüssigkeitsfilms in dem Folienbeutel 3 im Bereich der Lauffläche 11 dargestellt ist.

In Figur 4 ist erkennbar, dass ein Abstandswert des Stauabstands A nicht entlang der gesamten Länge der Stauflächen 5, 7 gleich ist. Vielmehr weist hier die zweite Staufläche 7 in einem Bereich vor dem Bremselement 9 eine Stufe 44 auf, an der sich Stauabstand A ändert, nämlich von einem ersten, größeren Abstandswert vor der Stufe 44 zu einem zweiten, kleineren Abstandswert hinter der Stufe 44.

Um die Beutelspannung insbesondere durch geeignete Ansteuerung des Spannelements 17 regeln zu können, ist bevorzugt eine Messeinrichtung 45 vorgesehen, die eingerichtet ist zur Ermittlung der Schichtdicke D, sei es direkt oder indirekt, wobei es genügt, dass die Messeinrichtung 45 eine Messgröße bereitstellt, auf die zur Einstellung einer homogenen und zeitlich konstanten Schichtdicke D geregelt werden kann, ohne dass hierzu die Schichtdicke D selbst explizit bestimmt werden muss.

Die Messeinrichtung 45 ist bevorzugt eingerichtet, um die Schichtdicke D mittels Druckmessung, optisch, elektrisch oder elektronisch, und/oder durch Erfassen einer Strömungsgeschwindigkeit des flüssigen Mediums in dem Folienbeutel 3 zu ermitteln.

Die Messeinrichtung 45 ist vorzugsweise mit der Steuereinrichtung 31 wirkverbunden, sodass die durch die Messeinrichtung 45 erfasste Messgröße in der Steuereinrichtung 31 zur Verfügung steht.

Bei dem in Figur 4 dargestellten Ausführungsbeispiel ist die Messeinrichtung 45 als Drucksensor 47 ausgebildet. Mittels des Drucksensors 47 ist insbesondere ein Druck als Maß für einen Strömungswiderstand für das flüssige Medium erfassbar, wobei bevorzugt der erfasste Druck zur Einstellung einer konstanten Schichtdicke D auf einen konstanten Wert eingeregelt werden kann.

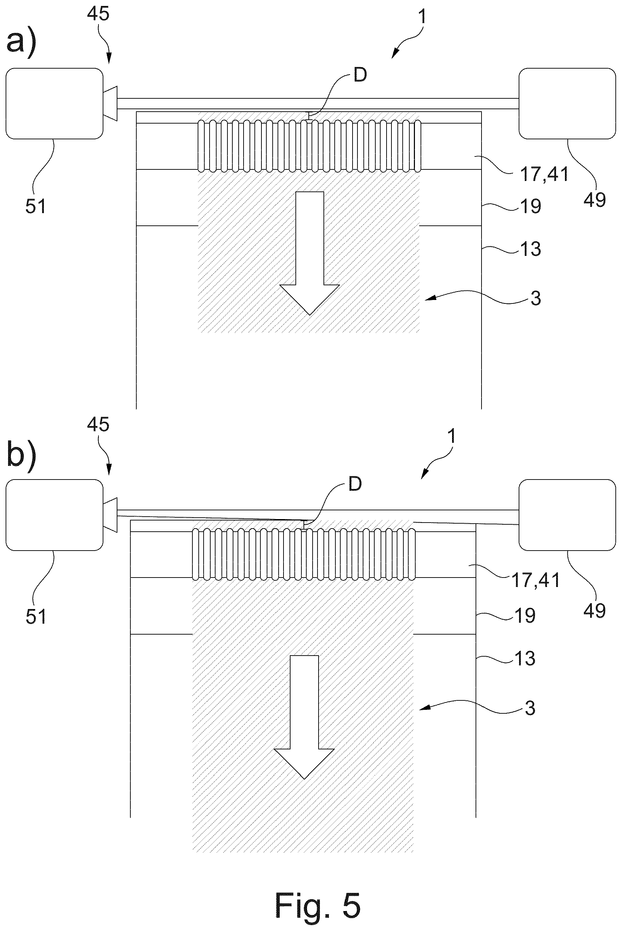

**Fig. 5** zeigt eine schematische Darstellung eines zweiten Ausführungsbeispiels der Vorrichtung 1. Dabei ist hier die Messeinrichtung 45 als optische Messeinrichtung ausgestaltet, welche die Schichtdicke D direkt erfasst, indem die Abschattung einer Lichtquelle 49 auf einem Sensor 51 erfasst wird. Die Lichtquelle 49 kann insbesondere eine LED oder Laserdiode sein. Der Sensor 51 kann insbesondere ein Fototransistor, eine Fotodiode, ein Zeilensensor, oder eine Kamera, insbesondere Zeilenkamera oder Matrixkamera, sein. Dabei ist der Sensor 51 im Bereich der Lauffläche 11 insbesondere der Lichtquelle 49 quer zur Transportrichtung, insbesondere senkrecht zur Transportrichtung, gegenüberliegend montiert, insbesondere derart, dass eine durch die Lichtquelle 49 erzeugte Lichtlinie sich entlang des Maximums der Lauffläche 11 und damit gerade in dem Bereich erstreckt, in dem die Schichtdicke D gemessen werden soll.

Erhöht sich die Schichtdicke D, wird eine Sensorfläche des Sensors 51 zunehmend abgeschattet. Erniedrigt sich umgekehrt die Schichtdicke D, wird mehr Licht von der Lichtquelle 49 zu dem Sensor 51 durchgelassen. Auf diese Weise kann insbesondere der höchste Punkt des Flüssigkeitsfilms detektiert werden. Für eine In-Line-Qualitätskontrolle ist dabei der maximale Wert der Schichtdicke D ausschlaggebend, da an diesem Punkt die Tiefendosisverteilung ihre größte Inhomogenität bei der Bestrahlung aufweist. Der derart gemessene Wert kann einem Regelalgorithmus zugeführt werden, welcher dann insbesondere die Spannrolle 41 ansteuert, um die Beutelspannung des Folienbeutels 3 entsprechend anzupassen.

Bei a) ist der Folienbeutel 3 in Figur 5 mit einer geringeren Schichtdicke D dargestellt; bei b) weist der Folienbeutel 3 insbesondere eine inhomogene und jedenfalls bereichsweise größere Schichtdicke D auf.

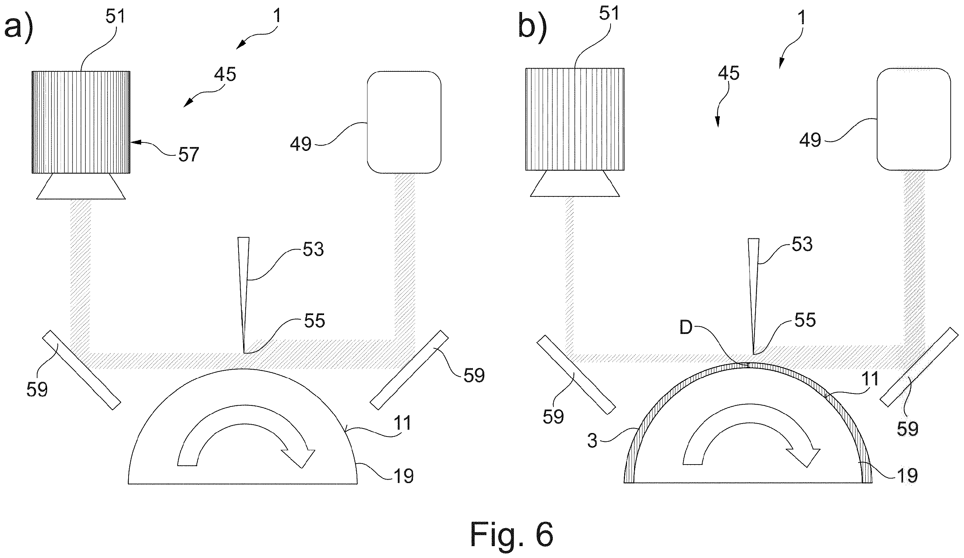

**Fig. 6** zeigt eine schematische Darstellung eines dritten Ausführungsbeispiels der Vorrichtung 1. Dabei ist hier die Messeinrichtung 45 eingerichtet zur optischen Interferenzmessung, mit einer Lichtquelle 49, die eingerichtet ist zur Emission kohärenter Lichtstrahlung, und einem Sensor 51 der eingerichtet ist, um ein Interferenzmuster zu erfassen. Die Lichtquelle 49 kann dabei insbesondere als Laser, insbesondere Laserdiode, ausgebildet sein. Der Sensor 51 ist bevorzugt eine Kamera oder ein Zeilensensor.

Im Bereich des Maximums der Lauffläche 11 und damit dem Bereich, in dem die Schichtdicke D gemessen werden soll, weist die Messeinrichtung 45 ein spaltbildendes Element 53 auf, hier einen Keil, das sich entlang der Lauffläche 11 quer zur Transportrichtung, insbesondere senkrecht zur Transportrichtung erstreckt, wobei ein spaltbildendes Ende 55, insbesondere ein spitzes Ende des spaltbildenden Elements 53 der Lauffläche 11 zugewandt und in einem Abstand zur Lauffläche 11 derart angeordnet ist, dass ein Spalt mit einer Größe gebildet ist, bei der Interferenz der durch die Lichtquelle 49 emittierten Strahlung in dem Sensor 51 feststellbar ist, selbst wenn kein Folienbeutel 3 in der Vorrichtung 1 angeordnet ist.

Die Breite des derart gebildeten Spalts wird dann definiert durch die fixe Anordnung des spaltbildenden Elements 53 einerseits sowie die Schichtdicke D des Flüssigkeitsfilms in dem Folienbeutel 3 andererseits.

Ein in dem Sensor 51 detektierbares Interferenzmuster 57 ist abhängig von der momentanen Breite des Spalts und damit direkt abhängig von der Schichtdicke D. Dabei zeigt a) eine Situation, in der kein Folienbeutel 3 in der Vorrichtung 1 angeordnet ist. Das Interferenzmuster 57 weist hier eine kleinere räumliche Periode auf.

Bei b) ist eine Situation dargestellt, in der ein Folienbeutel 3 mit einer endlichen Schichtdicke D des flüssigen Mediums auf der Lauffläche 11 angeordnet ist. Hierdurch ist der Spalt verengt, und das Interferenzmuster 57 weist eine größere räumliche Periode auf. Anhand der räumlichen Periode des Interferenzmusters 57 kann unmittelbar auf die Schichtdicke D rückgeschlossen werden.

Das Licht der Lichtquelle 49 wird hier durch zwei Umlenkspiegel 59 zweifach umgelenkt. Dies ist allerdings nicht zwingend erforderlich.

Eine Messung der Schichtdicke D kann auch anderweitig durch bildgebende Verfahren direkt oder indirekt, sowie durch Verfahren zur Bestimmung der Strömungsgeschwindigkeit des flüssigen Mediums innerhalb des Folienbeutels 3 erfasst werden.

Im Rahmen eines Verfahrens zum Erzeugen eines Flüssigkeitsfilms eines flüssigen Mediums in einem Folienbeutel 3 wird der Folienbeutel 3 in Transportrichtung durch die Vorrichtung 1 transportiert. Dabei wird ein gleichmäßiger, vorzugsweise eine zeitlich konstante Schichtdicke aufweisender Flüssigkeitsfilm erzeugt, indem zumindest der Bandantrieb 27 für das erste Transportband 13, oder ein gemeinsamer Bandantrieb für beide Transportbänder 13, 15, einerseits, sowie das Spannelement 17 andererseits unabhängig voneinander angesteuert werden.

## Patentansprüche

1. Vorrichtung (1) zur Erzeugung eines Flüssigkeitsfilms eines flüssigen Mediums in einem Folienbeutel (3), mit
- zwei voneinander durch einen Stauabstand (A) beabstandeten Stauflächen (5,7), zwischen denen der Folienbeutel (3) anordenbar und entlang einer Transportrichtung transportierbar ist,
- einem in Transportrichtung hinter den Stauflächen (5,7) angeordneten Bremselement (9), das eingerichtet ist, um den Folienbeutel (3) abzubremsen, und mit
- einer in Transportrichtung konvex gekrümmten Lauffläche (11), die derart eingerichtet und angeordnet ist, dass der durch das Bremselement (9) gebremste Folienbeutel (3) auf der Lauffläche (11) hinter dem Bremselement (9) aufgespannt werden kann, wobei
- zumindest eine erste Staufläche (5) der beiden Stauflächen (5,7) zumindest bereichsweise durch ein angetriebenes erstes Transportband (13) gebildet ist, das eingerichtet ist, um den Folienbeutel (3) in Transportrichtung zu fördern, wobei
- die Vorrichtung (1) in Transportrichtung hinter dem Bremselement (9) ein ansteuerbares Spannelement (17) aufweist, das eingerichtet ist, um den Folienbeutel (3) auf der Lauffläche (11) zwischen dem Bremselement (9) und dem Spannelement (17) aufzuspannen, und wobei
- das erste Transportband (13) oder ein Bandantrieb (27) für das erste Transportband (13) unabhängig von dem ansteuerbaren Spannelement (17) ansteuerbar ist.

2. Vorrichtung (1) nach Anspruch 1, wobei eine zweite Staufläche (7) der beiden Stauflächen (5,7) zumindest bereichsweise durch ein vorzugsweise angetriebenes zweites Transportband (15) gebildet ist.

3. Vorrichtung (1) nach einem der vorhergehenden Ansprüche, wobei das erste Transportband (13) auf einer ersten Stauplatte (23) relativ zu der ersten Stauplatte (23) verlagerbar angeordnet ist, wobei vorzugsweise das zweite Transportband (15) auf einer zweiten Stauplatte (25) relativ zu der zweiten Stauplatte (25) verlagerbar angeordnet ist, wobei bevorzugt der Folienbeutel (3) zwischen den Stauplatten (23,25) anordenbar und durch die Transportbänder (13,15) transportierbar ist.

4. Vorrichtung (1) nach einem der Ansprüche 2 oder 3, wobei
a) das erste Transportband (13) und das zweite Transportband (15) miteinander mechanisch bezüglich ihrer Laufgeschwindigkeit gekoppelt sind, oder
b) dem ersten Transportband (13) und dem zweiten Transportband (15) jeweils ein unabhängig ansteuerbarer Bandantrieb (27,29) zugeordnet ist, wobei vorzugsweise Laufgeschwindigkeiten für das erste Transportband (13) und das zweite Transportband (15) - bevorzugt elektronisch - einstellbar sind.

5. Vorrichtung (1) nach einem der vorhergehenden Ansprüche, wobei die zumindest eine der Stauflächen (5,7) derart relativ zu der anderen Staufläche (7,5) verlagerbar ist, dass der Stauabstand (A) einstellbar ist, wobei der Stauabstand (A) vorzugsweise
- entlang der Transportrichtung konstant, und/oder
- sich entlang der Transportrichtung verjüngend
einstellbar ist.

6. Vorrichtung (1) nach einem der vorhergehenden Ansprüche, wobei das Bremselement (9) als Bremsrolle (37) ausgebildet ist, die
- mit dem ersten Transportband (13) geschwindigkeitsgekoppelt,
- unabhängig von dem ersten Transportband (13) angetrieben, oder
- aktiv oder passiv gebremst
ist.

7. Vorrichtung (1) nach einem der vorhergehenden Ansprüche, wobei das Spannelement (17) als Spannrolle (41) ausgebildet ist, wobei die Vorrichtung (1) eine Steuereinrichtung (31) aufweist, die mit dem Spannelement (17) wirkverbunden und eingerichtet ist, um das Spannelement (17) in Abhängigkeit von einer bestimmten, vorzugsweise parametrierbaren Schichtdicke des zu erzeugenden Flüssigkeitsfilms
- drehmomentgesteuert, vorzugsweise drehmomentgeregelt, oder
- drehzahlgesteuert, vorzugsweise drehzahlgeregelt
anzusteuern.

8. Vorrichtung (1) nach einem der vorhergehenden Ansprüche, mit
- einer Temperiereinrichtung (33), die eingerichtet ist, um die Lauffläche (11) zu temperieren, und/oder mit
- einer Kühleinrichtung (35), die eingerichtet ist, um das Spannelement (17) zu kühlen.

9. Vorrichtung (1) nach einem der vorhergehenden Ansprüche, mit einer Messeinrichtung (45) zur Ermittlung einer Schichtdicke des zu erzeugenden Flüssigkeitsfilms, wobei die Messeinrichtung (45) vorzugsweise eingerichtet ist, um die Schichtdicke mittels Druckmessung, optisch, elektrisch oder elektronisch, und/oder durch Erfassen einer Strömungsgeschwindigkeit des flüssigen Mediums in dem Folienbeutel (3) zu ermitteln.

10. Anordnung (2) zur kontrollierten Exposition eines flüssigen Mediums in einem Folienbeutel (3) mit physikalischer Strahlung, umfassend eine Vorrichtung (1) nach einem der Ansprüche 1 bis 9 und eine Strahlungsquelle (4) physikalischer Strahlung, die angeordnet ist, um den Folienbeutel (3) im Bereich der konvexen Lauffläche (11) zu bestrahlen.

11. Verfahren zum Erzeugen eines Flüssigkeitsfilms eines flüssigen Mediums in einem Folienbeutel (3), wobei ein Folienbeutel (3) in Transportrichtung durch eine Vorrichtung (1) nach einem der Ansprüche 1 bis 9 transportiert wird, und wobei ein gleichmäßiger, vorzugsweise eine zeitlich konstante Schichtdicke aufweisender Flüssigkeitsfilm erzeugt wird, indem zumindest ein Bandantrieb (27) für das erste Transportband (13) und das Spannelement (17) unabhängig voneinander angesteuert werden.

## Claims

1. Device (1) for generating a liquid film of a liquid medium in a film bag (3), with
- two accumulating surfaces (5, 7) spaced apart by an accumulating distance (A), between which the film bag (3) can be arranged and transported along a transport direction,
- a braking element (9) arranged downstream of the accumulating surfaces (5, 7) in the transport direction, which braking element (9) is arranged to brake the film bag (3), and with
- a running surface (11) which is convexly curved in the transport direction and is set up and arranged in such a way that the film bag (3) braked by the braking element (9) can be spanned on the running surface (11) behind the braking element (9), wherein
- at least a first accumulating surface (5) of the two accumulating surfaces (5, 7) is formed at least in regions by a driven first conveyor belt (13) which is set up to convey the film bag (3) in the transport direction, wherein
- the device (1) has, downstream of the braking element (9) in the transport direction, a controllable tensioning element (17) which is set up to span the film bag (3) on the running surface (11) between the braking element (9) and the tensioning element (17), and wherein
- the first conveyor belt (13) or a belt drive (27) for the first conveyor belt (13) can be controlled independently of the controllable tensioning element (17).

2. Device (1) according to claim 1, wherein a second accumulating surface (7) of the two accumulating surfaces (5, 7) is formed at least in regions by a preferably driven second conveyor belt (15).

3. Device (1) according to one of the preceding claims, wherein the first conveyor belt (13) is arranged on a first accumulating plate (23) so as to be displaceable relative to the first accumulating plate (23), wherein preferably the second conveyor belt (15) is arranged on a second accumulating plate (25) so as to be displaceable relative to the second accumulating plate (25), wherein preferably the film bag (3) can be arranged between the accumulating plates (23, 25) and can be transported by the conveyor belts (13, 15).

4. Device (1) according to one of claims 2 or 3, wherein
a) the first conveyor belt (13) and the second conveyor belt (15) are mechanically coupled to each other with respect to their running speed, or
b) the first conveyor belt (13) and the second conveyor belt (15) are each assigned an independently controllable belt drive (27, 29), wherein preferably running speeds for the first conveyor belt (13) and the second conveyor belt (15) are adjustable - preferably electronically.

5. Device (1) according to one of the preceding claims, wherein the at least one of the accumulating surfaces (5, 7) is displaceable relative to the other accumulating surface (7, 5) in such a way that the accumulating distance (A) is adjustable, wherein the accumulating distance (A) is preferably adjustable
- constant along the transport direction, and/or
- tapering along the transport direction.

6. Device (1) according to one of the preceding claims, wherein the braking element (9) is configured as a brake roller (37) which is
- speed-coupled with the first conveyor belt (13),
- driven independently of the first conveyor belt (13), or
- actively or passively braked.

7. Device (1) according to one of the preceding claims, wherein the tensioning element (17) is configured as a tensioning roller (41), wherein the device (1) has a control device (31) which is operatively connected to the tensioning element (17) and is set up to control the tensioning element (17) as a function of a specific, preferably parameterizable layer thickness of the liquid film to be produced
- torque-controlled, preferably torque-regulated, or
- speed-controlled, preferably speed-regulated.

8. Device (1) according to one of the preceding claims, with
- a temperature control device (33) which is arranged to temper the running surface (11), and/or with
- a cooling device (35) arranged to cool the tensioning element (17).

9. Device (1) according to one of the preceding claims, having a measuring device (45) for determining a layer thickness of the liquid film to be produced, wherein the measuring device (45) preferably being set up to determine the layer thickness by means of pressure measurement, optically, electrically or electronically, and/or by detecting a flow rate of the liquid medium in the film bag (3).

10. Assembly (2) for controlled exposure of a liquid medium in a film bag (3) to physical radiation, comprising a device (1) according to one of claims 1 to 9 and a radiation source (4) of physical radiation arranged to irradiate the film bag (3) in the region of the convex running surface (11).

11. Method for generating a liquid film of a liquid medium in a film bag (3), wherein a film bag (3) is transported in the transport direction through a device (1) according to one of claims 1 to 9, and wherein a uniform liquid film, preferably having a temporally constant layer thickness, is produced by controlling at least one belt drive (27) for the first conveyor belt (13) and the tensioning element (17) independently of one another.

## Revendications

1. Dispositif (1) de production d'un film liquide d'un milieu liquide dans un sac en pellicule (3), avec
- deux surfaces d'accumulation (5, 7) espacées l'une de l'autre par un espace d'accumulation (A) entre lesquelles le sac en pellicule (3) peut être agencé et transporté le long d'un sens de transport,
- un élément de freinage (9), agencé dans le sens de transport derrière les surfaces d'accumulation (5, 7), qui est conçu pour ralentir le sac en pellicule (3), et avec
- une surface de roulement (11) incurvée d'une manière convexe dans le sens de transport, qui est conçue et agencée d'une manière telle que le sac en pellicule (3) freiné par l'élément de freinage (9) peut être tendu sur la surface de roulement (11) derrière l'élément de freinage (9), dans lequel
- au moins une première surface d'accumulation (5) des deux surfaces d'accumulation (5, 7) est formée au moins par endroits par une première bande transporteuse entraînée (13) qui est conçue pour transporter le sac en pellicule (3) dans le sens de transport, dans lequel
- le dispositif (1) présente un élément tendeur actionnable (17) qui est conçu pour tendre le sac en pellicule (3) sur la surface de roulement (11) entre l'élément de freinage (9) et l'élément tendeur (17), et dans lequel
- la première bande transporteuse (13) ou un entraînement de bande (27) pour la première bande transporteuse (13) peut être actionnée indépendamment de l'élément tendeur (17) actionnable.

2. Dispositif (1) selon la revendication 1, dans lequel une deuxième surface d'accumulation (7) des deux surfaces d'accumulation (5, 7) est formée au moins par endroits par une deuxième bande transporteuse (15) de préférence actionnée.

3. Dispositif (1) selon l'une des revendications précédentes, dans lequel la première bande transporteuse (13) est agencée d'une manière déplaçable sur une première plaque d'accumulation (23) par rapport à la première plaque d'accumulation (23), dans lequel, de préférence, la deuxième bande transporteuse (15) est agencée d'une manière déplaçable sur une deuxième plaque d'accumulation (25) par rapport à la deuxième plaque d'accumulation (25), dans lequel, de préférence, le sac en pellicule (3) peut être agencé entre les plaques d'accumulation (23, 25) et peut être transporté par les bandes transporteuses (13, 15).

4. Dispositif (1) selon l'une des revendications 2 ou 3, dans lequel
a) la première bande transporteuse (13) et la deuxième bande transporteuse (15) sont couplées ensemble mécaniquement par rapport à leur vitesse de roulement, ou
b) à chaque fois un entraînement de bande (27, 29) actionnable indépendamment peut être affecté à la première bande transporteuse (13) et à la deuxième bande transporteuse (15), dans lequel, de préférence, des vitesses de roulement peuvent être réglées - de préférence, électroniquement - pour la première bande transporteuse (13) et la deuxième bande transporteuse (15).

5. Dispositif (1) selon l'une des revendications précédentes, dans lequel la au moins une des surfaces d'accumulation (5, 7) est déplaçable d'une manière telle par rapport à l'autre surface d'accumulation (7, 5) que l'espace d'accumulation (A) peut être réglé, dans lequel l'espace d'accumulation (A) peut être réglé de préférence
- d'une manière constante le long du sens de transport, et / ou
- en se rétrécissant le long du sens de transport.

6. Dispositif (1) selon l'une des revendications précédentes, dans lequel l'élément de freinage (9) est conçu comme un rouleau de freinage (37), qui est
- couplé en vitesse à la première bande transporteuse (13),
- actionné indépendamment de la première bande transporteuse (13), ou
- freiné activement ou passivement.

7. Dispositif (1) selon l'une des revendications précédentes, dans lequel l'élément tendeur (17) est conçu comme un galet tendeur (41), dans lequel le dispositif (1) présente un dispositif de commande (31) qui est en liaison active avec l'élément tendeur (17) et agencé pour commander l'élément tendeur (17) en fonction d'une certaine épaisseur de couche, de préférence paramétrable, du film liquide à produire
- avec commande en fonction du couple, de préférence, réglage en fonction du couple, ou
- avec commande en fonction de la vitesse, de préférence, réglage en fonction de la vitesse.

8. Dispositif (1) selon l'une des revendications précédentes, avec
- un dispositif de régulation de température (33) qui est conçu pour réguler la température autour de la surface de roulement (11), et/ou avec
- un dispositif de refroidissement (35) qui est conçu pour refroidir l'élément tendeur (17).

9. Dispositif (1) selon l'une des revendications précédentes, avec un dispositif de mesure (45) pour détecter une épaisseur de couche du film liquide à produire, dans lequel le dispositif de mesure (45) est conçu, de préférence, pour détecter l'épaisseur de couche au moyen de la mesure de pression, optiquement, électriquement ou électroniquement, et/ou par détection d'une vitesse d'écoulement du milieu liquide dans le sac en pellicule (3).

10. Dispositif (2) pour l'exposition contrôlée d'un milieu liquide dans un sac en pellicule (3) à l'aide d'un rayonnement physique, comprenant un dispositif (1) selon l'une des revendications 1 à 9 et une source de rayonnement (4) de rayonnement physique qui est agencée pour irradier le sac en pellicule (3) dans la zone de la surface de roulement convexe (11).

11. Procédé de production d'un film liquide d'un milieu liquide dans un sac en pellicule (3), dans lequel un sac en pellicule (3) est transporté dans le sens de transport par un dispositif (1) selon l'une des revendications 1 à 9, et dans lequel est produit un film de liquide uniforme, présentant, de préférence, une épaisseur de couche constante dans le temps, tandis qu'au moins un entraînement de bande (27) pour la première bande transporteuse (13) et l'élément tendeur (17) sont commandés indépendamment l'un de l'autre.
